# FASCICULE DE BREVET EUROPEEN

(11) **EP 1 812 089 B1**
(45) Date de publication et mention de la délivrance du brevet: **23.01.2013**
(21) Numéro de dépôt: 05812196.3
(22) Date de dépôt: 24.10.2005
(51) Int. Cl.: A61L 27/46, A61L 27/36

(54) **COMPOSITION DE COMBLEMENT OSSEUX**
ZUSAMMENSETZUNG ZUM AUFFÜLLEN EINES KNOCHENDEFEKTS
COMPOSITION FOR FILLING A BONE DEFECT

(30) Priorité: 22.10.2004 FR 0411271
(43) Date de publication de la demande: 01.08.2007
(73) Titulaire: TBF - Banque de Tissus, 69500 Bron (FR)
(72) Inventeur: BARNOUIN, Laurence, F-38460 Venerieu (FR); LAGANIER, Laurent, F-69390 Vernaison (FR)
(74) Mandataire: Tripoz, Inès
(86) Numéro de dépôt international: PCT/FR2005/002651
(87) Numéro de publication internationale: WO 2006/045944

(56) Documents cités:
- US-A- 5 571 183
- US-A1- 2002 110 541
- RUSKIN J D ET AL: "Alveolar ridge repair in a canine model using rhTGF-beta 1 with barrier membranes." CLINICAL ORAL IMPLANTS RESEARCH. APR 2000, vol. 11, no. 2, avril 2000 (2000-04), pages 107-115, XP002335964 ISSN: 0905-7161 cité dans la demande
- PIETRZAK W S ET AL: "Calcium sulfate bone void filler: a review and a look ahead." THE JOURNAL OF CRANIOFACIAL SURGERY. JUL 2000, vol. 11, no. 4, juillet 2000 (2000-07), pages 327-333 ; dis, XP009050617 ISSN: 1049-2275 cité dans la demande

## Description

Le comblement de cavités osseuses est un problème fréquent en chirurgie orthopédique. Différentes techniques chirurgicales et une gamme de produits sont potentiellement utilisables par les chirurgiens en fonction des défauts à combler et de leur origine, qui peuvent être dus à une chirurgie reconstructrice et à la pose de plaques, vis ou prothèses ou pathologiques et dus à des tumeurs par exemple.

Des produits de comblement peuvent être d'origine humaine, animale, minérale ou de synthèse. Tous ces matériaux doivent être biocompatibles (c'est-à-dire non toxiques, non immunogènes, et non carcinogènes), ils doivent favoriser ou induire une repousse osseuse, être résorbables à moyen terme, et être stockables et disponibles. Le choix du matériau est influencé par le volume à combler, le lieu, les conditions locales, et le but recherché.

Cette invention concerne ainsi le domaine des compositions destinées à l'amélioration de la réparation tissulaire osseuse et au comblement de défauts ou défects osseux.

Le tissu osseux est constitué d'une matrice de tissu conjonctif sur laquelle se fixent les éléments minéraux qui en assurent la rigidité. On distingue deux types de structures : l'os compact et l'os spongieux, formé de travées osseuses entre lesquelles se situe du tissu hématopoïétique ou graisseux. Dans les deux cas, l'os est formé de lamelles superposées, de quelques microns d'épaisseur, dont l'orientation suit celle des fibres de collagène.

La répartition de ces deux types d'os est différente selon le site squelettique : l'os spongieux est majoritaire au niveau des corps vertébraux, de l'épiphyse radiale et du calcaneum, alors que l'os cortical se trouve au niveau des arcs vertébraux postérieurs, des diaphyses des os longs, du col fémoral.

Ce tissu conjonctif spécialisé est constitué d'une matrice organo-minérale et de cellules. La matrice du tissu osseux est formée à 70 % de la phase minérale, 20 % de constituants organiques et de 10 % d'eau. Cette répartition pondérale n'est qu'approximative, puisque la nature des éléments constitutifs de l'os reste sensiblement constante, alors que leur proportion peut varier considérablement selon la nature de l'os, le sexe, l'alimentation et l'âge.

Le composant minéral osseux comprend les éléments suivants :
Calcium (% massique : 36,7 %), Phosphore (16,0 %), Carbonate (8,0 %), Sodium (0,77 %), Magnésium (0,46 %) et Fluor (0,04 %).

La phase cristalline de l'os s'apparente à l'hydroxyapatite (Ca₁₀ (PO₄)₆ (OH)₂) dont la maille est hexagonale. L'apatite est une structure hôte pouvant incorporer un nombre important d'éléments chimiques ou laisser apparaître des lacunes.

La matrice organique est formée pour 90 % de collagène de type 1, pour 10 % d'autres protéines non collagéniques, en grande partie de l'ostéocalcine et de l'ostéonectine. Cette matrice est dégradée et synthétisée respectivement par deux types de cellules : les ostéoclastes et les ostéoblastes. L'activité de ces deux cellules contrôle le processus dynamique dit de « remodelage » qui, tout au long de la vie, renouvelle les tissus de la matrice osseuse et permet de conserver leurs propriétés biomécaniques.

Il existe deux principaux types de cellules osseuses : les ostéoblastes et les ostéoclastes.

Les ostéoblastes sont à l'origine de la genèse du tissu osseux. Ils se situent à la surface de tissu osseux, le plus souvent en bordure d'un tissu en cours de formation : tissu ostéoïde.

La différenciation des ostéoblastes correspond à un processus complexe qui met en jeu des interactions multiples entre les cellules et la matrice. Après une étape de prolifération initiale des cellules, précurseurs, l'ostéoblaste acquiert progressivement les caractéristiques d'une cellule fonctionnelle différenciée. Sa fonction essentielle est de synthétiser et de minéraliser la matrice organique osseuse constitué essentiellement de collagène, de protéines non collagéniques et de facteurs de croissance.

De plus, les cellules de la lignée ostéoblastique jouent un rôle important dans le contrôle du remodelage osseux, d'une part par leur capacité à synthétiser de nombreux facteurs de croissance, d'autre part en tant que cellules cibles des hormones contrôlant la différenciation des ostéoclastes.

La formation de l'os dépend essentiellement du nombre d'ostéoblastes, plutôt que de l'activité de chacun d'entre eux. En outre, il est mis en évidence l'importance de l'étape de prolifération cellulaire à partir des précurseurs ostéoblastiques dans le contrôle de la formation osseuse normale et pathologique, et il est souligné le rôle primordial des facteurs de croissance affectant le recrutement et la prolifération des ostéoblastes.

Une fois le tissu osseux formé, les ostéoblastes s'y retrouvent piégés. Ces cellules sont alors nommées Ostéocytes.

Les ostéoclastes sont responsables du processus de résorption osseuse, ce sont des cellules multinucléées, très volumineuses. Les ostéoclastes se situent à la surface de l'os au niveau de zones en résorption. La résorption osseuse est rendue possible par un contenu enzymatique important. La dissolution de la fraction minérale s'effectue par un abaissement local du pH au voisinage de 5, dû à la libération extracellulaire de protons sous l'action de l'anhydrase carbonique. La dégradation de la matrice organique se réalise par clivage enzymatique par action de cathépsines et d'hydrolases.

Les deux types de cellules osseuses, ostéoclastes et ostéoblastes, proviennent respectivement des cellules souches hématopoïétiques et des cellules stromales de la moelle osseuse.

Trois mécanismes contribuent au dépôt de l'os après une greffe osseuse : l'ostéogénèse, l'ostéoconduction et l'ostéoinduction.
- l'ostéogénèse se traduit par la formation d'os à partir des ostéoblastes implantés.
- l'ostéoconduction comporte 3 étapes : l'infiltration du greffon par les ostéoblastes de l'hôte, leur prolifération, puis la néoformation d'os dans un environnement favorable (échaffaudage d'os autologue et matrice d'Hydroxyapatite).
- l'ostéoinduction correspond à la néoformation d'os à partir des cellules mésenchymateuses du receveur qui se différencient en ostéoblastes sous l'influence des facteurs de croissance présents dans la matrice.

Quatre éléments essentiels sont nécessaires pour permettre la régénération osseuse :
- la matrice ostéoconductrice,
- les facteurs ostéoinducteurs c'est-à-dire les agents chimiques qui induisent les différentes étapes de la réparation osseuse,
- les cellules ostéogéniques qui peuvent se différencier et faciliter la réparation osseuse,
- l'intégrité structurale.

Dans le cas d'une autogreffe spongieuse, la revascularisation complète est effectuée en deux à trois semaines. Les ostéoblastes du greffon du site receveur déposent directement du tissu ostéoïde sur les travées, la consolidation greffe-os est acquise en cinq à six semaines. Dans le cas d'une greffe corticale, une phase préliminaire de résorption ostéoclastique est nécessaire et la consolidation n'est obtenue qu'à partir du troisième mois.

Une autogreffe possède donc les quatre éléments nécessaires à la régénération osseuse.

Dans le cas d'une allogreffe, l'os allogénique, n'a en lui même, aucune propriété ostéogénique, c'est-à-dire qu'il est incapable à lui seul de susciter la formation d'os nouveau. Sa propriété essentielle est d'être un guide à la repousse osseuse provenant du lit osseux dans lequel il est placé. L'étendue de la surface de contact greffe hôte est déterminante dans la rapidité de la pénétration de la greffe. Les greffes totalement encastrées comme dans une cavité osseuse ont un environnement plus favorable à l'ostéogénèse que celles simplement apposées.

Une allogreffe possède donc seulement deux des éléments nécessaires à la régénération osseuse.

Les pathologies de l'os nécessitant des compositions de comblement selon l'invention sont les pathologies comme les tumeurs osseuses

Les tumeurs bénignes, qui sont beaucoup plus fréquentes que les tumeurs malignes, sont de différents types histologiques (ostéome ostéoïde, exostose, fibrome...) et peuvent aboutir à des greffes de comblement.

Les tumeurs malignes dont le traitement est celui d'un cancer primitif ne fait pas ou très peu appel à des greffes de comblement.

De nombreuses interventions en chirurgie orthopédique (ou dentaire) nécessitent la pose de plaques, de vis, de prothèses. Afin de faciliter l'intégration de ces dispositifs dans le site osseux, les interstices peuvent être comblés à l'aide d'une greffe osseuse ou d'un substitut osseux.

A l'heure actuelle, c'est ce type d'interventions qui nécessitent le plus les compositions de comblements osseux.

Le produit de comblement osseux idéal est un biomatériau ayant des propriétés proches de l'os, pouvant être à la fois sûr, efficace et disponible :
La sécurité d'un biomatériau repose sur sa biocompatibilité, c'est-à-dire sur l'absence de toxicité de ce biomatériau et des produits de dégradation. L'évaluation de cette sécurité est essentielle et doit être effectuée avant l'étude de sa biofonctionnalité chez l'animal. Elle repose sur l'application de normes reconnues. Outre les études de mutagénicité et de carcinogénicité, la recherche des réactions de l'hôte vis-à-vis du biomatériau nécessite une analyse approfondie de la réaction inflammatoire (présence de cellules géantes, interposition de tissus conjonctifs entre biomatériau et tissus de l'hôte...).

L'efficacité d'un substitut osseux repose sur ses résultats cliniques à court, moyen et long termes.

L'étude des qualités d'ostéoconduction et éventuellement d'ostéoinduction du biomatériau est intéressant pour prédire son l'efficacité clinique.

L'ostéoconduction correspond à la « propriété passive d'un matériau à recevoir la repousse osseuse, par invasion vasculaire et cellulaire à partir du tissu osseux receveur au contact du matériau ». L'ostéoconduction est en partie dépendante de la taille des pores du biomatériau.

L'ostéoinduction est la capacité à favoriser la repousse osseuse par induction du métabolisme osseux du site osseux receveur.

La disponibilité d'un substitut osseux suppose qu'il puisse être fabriqué en quantité suffisante pour répondre à toutes les demandes, qu'il puisse se conserver suffisamment longtemps sans dégradation significative de ses propriétés essentielles et que ses conditions de conservation soient les plus simples, à la portée de tout service de chirurgie osseuse.

Les comblements osseux utilisés aujourd'hui sont de nature et de compositions diverses et variées. Deux types principaux de comblements osseux sont distingués : les greffes osseuses et les substituts osseux.

Le terme de greffe osseuse désigne un apport de tissu osseux libre, vivant ou mort. Deux catégories existent : les autogreffes et les allogreffes à l'exclusion des produits de comblement osseux composés contenant de l'os et un autre produit.

L'autogreffe est le matériau de comblement le plus ancien actuellement encore utilisé par les chirurgiens orthopédiques. L'autogreffe est le matériau de référence des substituts osseux. Par définition l'autogreffe est prise sur le patient au niveau d'un site donneur (hanche, crâne...) et est placée dans le même temps opératoire à l'endroit où le comblement est nécessaire. Elle est le plus ostéogénique des matériaux de comblement: ostéoinductrice et ostéoconductrice.

L'allogreffe désigne un greffon pris sur un humain qui n'est pas le receveur.

Différentes approches sont à ce jour employées pour la conservation des allogreffes. La greffe peut être soit simplement congelée, soit lyophilisée soit lavée. Une stérilisation complémentaire est conseillée par le biais de moyens physiques (irradiation ionisante), chimiques (oxyde d'éthylène) ou par la chaleur. Le but de ces traitements étant de diminuer l'antigénicité du tissu osseux sans trop en altérer les propriétés biologiques et mécaniques.

Lorsqu'une greffe n'est pas possible ou souhaitable, les chirurgiens ont recours aux substituts osseux.

Parmi les substituts osseux connus on peut citer les xénogreffes qui désignent une greffe d'origine animale. La plus courante est préparée à partir d'os de boeuf adulte.

Les difficultés liées à la préparation de tels produits résident dans l'obtention d'une sécurité biologique indispensable et la conservation de l'intégrité de la structure biphasique de l'os (collagénique et minérale).

Les dérivés du corail sont également utilisés, ils ont une structure totalement poreuse qui favorise la pénétration osseuse. Ils sont utilisés depuis longtemps.

Les ciments phosphocalciques ou ioniques forment une nouvelle classe de substituts osseux caractérisés par une prise et un durcissement en milieu humide. Cependant leur objectif est essentiellement le scellement de prothèses et non le comblement osseux.

Les céramiques de phosphate de calcium sont également utilisées car les cristaux d'apatites biologiques, constituants principaux des os et des dents, appartiennent à la famille des phosphates de calcium. Ils forment la fraction minérale de l'os et pourraient agir comme des précurseurs lors de la minéralisation.

Les substituts minéraux, à base de sulfate de calcium voire de résine synthétique sont également disponibles.

D'autres substituts osseux existent, Ils sont composés de deux phases (os allogénique et autre produit), comme par exemple des mélanges de sulfate de calcium et os déminéralisé, de glycérol et os déminéralisé, de gélatine et os déminéralisé, de hyaluronate de sodium et d'os déminéralisé, ou d'un copolymère et d'os déminéralisé, sous forme de pâte préparée extemporanément ou de pâte prête à l'emploi.

Des problèmes demeurent car une composition de comblement osseux, matériau idéal de comblement permettant de faire face à toutes les situations n'existe pas. Un comblement ne sera pas envisagé de la même façon après exérèse d'une tumeur, après délabrement traumatique d'un membre que ce soit en phase aiguë ou au stade de pseudarthrose, après descellement d'une prothèse, après une ostéotomie d'addition, ou lors du traitement de scolioses étendues.

Les matériaux de comblement sont d'un intérêt pratique important car le comblement de défects osseux est nécessaire dans de multiples situations comme par exemple, l'évidement tumoral, pour le comblement après évidement tumoral, le comblement de défects spongieux non tumoraux et non septiques, la reconstruction d'os cortical et la chirurgie du corps vertébral.

Ces indications extrêmement variées demandent des propriétés mécaniques étendues et des consistance et présentation diverses.

De nombreuses publications et brevets décrivent des compositions telles que précédemment citées. Par exemple, on connaît de US-B-6030635 une composition à base d'hydrogels choisis parmi le hyaluronate de sodium et des chitosanes permettant de réaliser des compositions ne comportant que 1 à 3% de gel, et de WO-A-9639203 des compositions de collagène comportant des particules d'os déminéralisé.

On connaît de US2002/0110541 une composition comprenant du sulfate de calcium, une solution de mélange et une substance plastifiante constituée par un dérivé cellulosique, comme la carboxyméthyl cellulose qui forme un hydrogel mucoadhésif et biodégradable capable de former un produit de comblement présentant une résistance mécanique importante et pouvant être utilisé seul, voir paragraphe « robustness » dans la description.

On connaît de Ruskin J.D. et al. dans Clinical oral implants research, april 200, vol 11, 2, pp107-115 la préparation d'une composition comprenant du carbonate de calcium, de l'hydroxyéthylamidon et un facteur de croissance pour tester l'ostéogénèse et la régénération osseuse dans des lésions osseuses buccales.

On connaît de Pietrrzak W.S. et al., dans The journal of craniofacial surgery, july 2000, vol 11, 4, pp 327-333, l'intérêt de l'utilisation du sulfate de calcium comme produit de comblement in-vivo.

Cependant aucun des produits actuellement décrit et/ou commercialisé ne permet de satisfaire à l'ensemble des critères ci-dessus développés.

La présente invention a permis de réaliser des compositions permettant de faire face à l'ensemble des situations décrites en conservant des propriétés satisfaisant l'ensemble des critères.

En effet, idéalement ces compositions de comblement osseux doivent pour en faciliter leur mise en oeuvre se présenter au moment de leur utilisation, c'est-à-dire au cours des interventions chirurgicales, sous forme de pâte malléable sans nécessiter de réchauffage, éventuellement moulable pour permettre une mise en oeuvre après mise en forme, non adhésive sur les instruments et sur les gants, mais suffisamment cohésive et capable d'adhérer sur les tissus des sites sur lesquels la pâte sera appliquée. Elles doivent également être ostéogéniques.

La présente invention concerne donc une composition de comblement osseux sous forme de pâte malléable caractérisée en ce qu'elle comprend de la poudre d'os allogénique, du sulfate de calcium, un liant choisi parmi de l'amidon ou un dérivé d'amidon et une solution de mélange.

Selon l'invention le dérivé d'amidon est choisi parmi l'hydroxyéthyl amidon, le carboxyméthyl amidon, l'amidon glycolique, l'amidon glycérol, l'amidon pregélatinisé.

Elle concerne également la composition de comblement osseux selon l'invention caractérisée en ce que le dérivé d'amidon est l'hydroxyéthyl amidon.

Selon l'invention, le sulfate de calcium est choisi parmi le sulfate de calcium dihydraté, anhydre ou hémihydraté, dont la forme cristalline caractérisée peut être alpha, bêta ou les deux. Il est de préférence le sulfate de calcium hémihydraté.

La poudre d'os allogénique selon l'invention, est choisie parmi les poudres d'os humain ou non, déminéralisée ou non, ayant subi un traitement de viroinactivation, de granulométrie comprise entre 200 et 1600 µm.

Elle est de préférence une poudre d'os humain, ayant subi un traitement de viroinactivation de granulométrie comprise entre 200 et 1600 µm.

Selon l'invention, la solution de mélange est une solution aqueuse compatible physiologiquement choisie parmi un soluté isotonique, du sérum physiologique ou un liquide physiologique comme du sérum ou du sang.

Selon les applications, la solution aqueuse pourra être tamponnée.

Celle-ci sera additionnée au mélange en fonction de la consistance de la composition finale souhaitée, à savoir une composition sous forme de pâte malléable, injectable ou susceptible d'être moulée.

Selon l'invention, la poudre d'os allogénique sera incorporée dans des proportions comprises entre 0,1 et 80% v/v, le sulfate de calcium dans des proportions de 50 à 300% m/o, l'hydroxyéthyl amidon de 0,1 à 20% v/v et la solution de mélange de 0,3 à 80% v/v.

Le sulfate de calcium existe sous trois formes : dihydraté (CaSO_{4·}2H₂O), hémihydraté (CaSO₄-1/2H₂O) et anhydre(CaSO₄).

Le sulfate de calcium dihydraté est le constituant principal du Gypse.

C'est à partir de ce minerai que le plâtre de Paris est fabriqué. Après extraction, le Gypse est concassé, broyé et séché. Soumis ensuite à une « cuisson » (entre 100 et 200°C), il se déshydrate partiellement et donne naissance au plâtre de Paris : sulfate de calcium hémihydraté.

Selon le mode de cuisson utilisé, deux formes de plâtres sont principalement recherchées :
- le plâtre hémihydraté Alpha, obtenu par cuisson sous pression en autoclave.
- le plâtre hémihydraté Bêta, obtenu en four à lits fluidisés ou en four à flammes directes.

Les sulfates de calcium dihydraté et hémihydraté ont une différence de solubilité à une température de 20°C. Ainsi l'ajout d'eau à une poudre de sulfate de calcium hémihydraté va engendrer la formation de sulfate de calcium dihydraté qui va précipiter pour donner une pierre dure. La prise du plâtre traduit la cristallisation du gypse, elle s'accompagne d'une élévation de température caractéristique qui peut atteindre 37°C.

La quantité d'eau utilisée pour la prise du plâtre agit sur 2 niveaux :
- l'eau de réhydratation qui retransforme le plâtre en gypse ou eau de cristallisation.
- l'eau en excédant qui contribue à fixer la valeur de porosité et qui s'élimine au séchage.

L'implantation de plâtre de Paris dans de l'os ou des tissus ne produit pas de réactions inflammatoires, ce dernier est bien accepté par le corps, mais le plâtre de Paris seul ne stimule pas mais n'inhibe pas la formation de l'os et il est absorbé et déplacé du site d'implantation très rapidement et l'os se reconstruit.

La poudre d'os permet la repousse osseuse au niveau du site comblé, elle est constituée d'un mélange de poudre d'os calcifié et de poudre d'os déminéralisé. Elle apporte à la fois le calcium par la poudre d'os calcifié et les facteurs de croissance nécessaires à la néoformation osseuse par la poudre d'os déminéralisé.

L'os peut être d'origine allogénique et issu de la transformation de têtes fémorales ou d'os massifs d'origine humaine traités pour inactiver les virus, puis réduits sous forme de copeaux.

La poudre d'os calcifié peut ainsi être obtenue par broyage direct de copeaux d'os spongieux ou cortical.

La poudre d'os calcifié, a pour rôle d'apporter les minéraux nécessaires au processus de néoformation osseuse au niveau du site comblé, sans qu'il soit nécessaire d'apporter des sels minéraux en complément pour activer cette néoformation osseuse.

La poudre d'os déminéralisé est obtenue par broyage d'os et déminéralisation ou, par déminéralisation de fragments osseux qui sont ensuite broyés.

La poudre d'os déminéralisé apporte les facteurs de croissance favorisant la néoformation osseuse qui stimulent les cellules ostéoprogénitrices.

La combinaison des deux poudres d'os, c'est-à-dire poudre d'os calcifié et poudre d'os déminéralisé, permet la stimulation de la repousse osseuse par ostéoconduction et ostéoinduction.

La granulométrie des poudres d'os a un rôle important dans la viscosité et la consistance de la pâte et sera comprise entre 100 et 1600 µm, et de préférence, entre 200 et 500 µm pour la poudre d'os calcifié.

Par exemple les greffons PHOENIX® sont commercialisés par TBF, Banque de tissus peuvent être utilisés.

Le procédé de transformation des têtes fémorales en greffons PHOENIX® à pour finalité de nettoyer les travées osseuses et d'inactiver par des solvants chimiques les micro-organismes non détectés par l'examen sanguin. Cette inactivation a été validée par l'institut Pasteur Texcell sur des virus particulièrement résistants. Une étape du procédé permet également de se prémunir contre les agents transmissibles non conventionnels (prions).

Le procédé de transformation a été étudié pour ne pas altérer les propriétés mécaniques intrinsèques de l'os spongieux.

Le sulfate de calcium présente de nombreuses caractéristiques qui sont des avantages pour un substitut osseux, il est inorganique, ainsi tous les risques de transmission de virus ou de maladies sont évités, il est biocompatible, il est résorbable, il est ostéoconducteur, :il sert de support pour les vaisseaux sanguins, les cellules et permet la revascularisation, Il constitue une source de calcium, élément essentiel pour la reconstruction osseuse.

La forme hémihydratée du sulfate de calcium possède plus particulièrement des caractéristiques physiques favorables : il est moulable, facilement manipulable, il possède la capacité de durcir et il est possible de le mélanger à d'autres matériaux.

L'amidon est un sucre polyoside de formule brute (C₆H₁₀O₅)ₙ, n étant compris entre 100 et 20 000. La composition chimique de l'amidon varie suivant son origine végétale, mais tous les amidons renferment deux polysaccharides, *l'amylose* et *l'amylopectine.*

On le prépare industriellement à partir de la pomme de terre, du blé, du maïs et du riz. La plupart des amidons de maïs, de pomme de terre et de blé contiennent 70 à 85 % d'amylopectine et 13 à 30 % d'amylose.

L'hydroxyethylamidon est de l'amidon sur lequel sont greffés des groupements hydroxyethyl (C₂H₄ - OH). Ces groupements hydroxyethyl peuvent être attachés au glucose par les carbones situés en positions 2, 3 et 6.

L'hydroxyethylamidon est un produit pharmaceutique en solution injectable elle sert de soluté de remplissage et de substitut du plasma et ne forme pas un hydrogel.solide.

Il y a trois paramètres qui permettent d'identifier les différents types d'hydroxyethylamidon :
- Le poids moléculaire : l'hydroxyethyl amidon est une macromolécule dont le poids moléculaire peut aller de 200 000 à 450 000 daltons. L'hydroxyethylamidon est dégradé d'autant plus rapidement par les amylases sériques que son taux de substitution molaire est bas.
- Le degré de substitution : il s'agit du nombre de groupement hydroxyethyl pour 10 molécules de glucose. Ce degré de substitution influe sur le temps de dégradation du produit. Plus le degré de substitution est élevé, plus le temps de dégradation est important.
- Le rapport C2/C6 : il s'agit du rapport entre le nombre de groupements hydroxyethyl attachés au carbone en position 2 et en position 6. L'hydrolyse par l'amylase est plus retardée par une substitution sur le carbone en position 2, que sur celui en position 6. Ainsi plus ce rapport augmente, plus le temps de dégradation de la macromolécule sera important.

L'invention concerne également l'utilisation pour le comblement des défects osseux d'une composition de comblement osseux, caractérisée en ce qu'elle comprend de la poudre d'os allogénique, du sulfate de calcium, un liant choisi parmi l'amidon ou un dérivé d'amidon et une solution de mélange.

Elle concerne plus particulièrement l'utilisation ci-dessus décrite, caractérisée en ce que le liant est l'hydroxyéthyl amidon.

Selon l'invention, la composition sera utilisée soit sous forme de pâte plus ou moins liquide, c'est-à-dire malléable ou injectable, soit sous forme de pâte préalablement moulée.

Dans un mode de mise en oeuvre différent lors de comblement de défauts osseux de volumes importants par exemple après chirurgie tumorale, la composition selon l'invention pourra être employée elle-même comme un véhicule pour l'implantation de fragments d'os.

En fonction des utilisations particulières possibles pour la composition selon l'invention, la composition selon l'invention peut comprendre en outre un ou plusieurs principes actifs pharmaceutiques choisi par exemple parmi les agents antiviraux, les antibiotiques, les immunosuppressant et ou les agents antitumoraux.

Elle peut également comprendre des facteurs de croissance qui seront libérés in situ comme les « Bone Morphing Protein tel BMP 2 ou 7 pour l'os, favorisant la réparation des tissus cibles.

L'invention concerne également la composition selon l'invention sous forme de kit, à savoir un contenant comprenant une composition comprenant de la poudre d'os allogénique, du sulfate de calcium, un liant choisi parmi de l'amidon ou un dérivé d'amidon et un contenant comprenant la solution de mélange.

Lorsque les compositions doivent être moulées pour une utilisation particulière par le chirurgien, par exemple pour mettre en place des coins d'ostéotomie, alors le kit selon l'invention, comprend en outre des moules pour coins d'ostéotomie

Des principes actifs pharmaceutiques ou des facteurs de croissance pouvant être additionnés, soit au contenant comprenant la poudre d'os, soit en solution dans le contenant comprenant la solution de mélange.

L'invention sera mieux comprise à la lecture des exemples de réalisations suivants :

### EXEMPLE 1 : Fabrication de compositions selon l'invention

Pour fabriquer un volume final de composition de comblement osseux, d'environ 3 cc, il faut :
1 cc de poudre d'os Phoenix, obtenue à partir d'os déminéralisé ou non ayant subit un traitement assurant sa sécurité microbiologique (viro-inactivation)
4 g de sulfate de calcium
0,1 g d'hydroxyethylamidon
1,1 ml de sérum physiologique (solution aqueuse de NaCl 0,9 %)

Le taux d'hydroxyethylamidon peut varier en fonction de la qualité utilisée : ainsi, l'augmentation du poids moléculaire moyen et du taux de substitution font augmenter la viscosité du produit.

La granulométrie de la poudre d'os utilisée peut faire varier la quantité d'os pour un même volume de poudre et modifier ainsi les proportions adéquates des autres composants. (granulométrie habituellement utilisée : de 200 à 1600 µm).

Le sérum physiologique peut-être remplacé par un autre soluté physiologique. En particulier un tampon peut-être rajouté.

Différentes formulations sont réalisées.

| | Coin d'ostéotomie 10,8 ml | Pâte 16,5 ml | Fraction du volume final (v/v ou p/v) |
|---|---|---|---|
| Sérum physiologique | 4 ml | 6 ml | 20 à 80% |
| Poudre d'os (granulométrie 20 - 3000 µm) | 3,6 ml | 5,5 ml | 1 à 80% |
| Sulfate de calcium | 14,4 g | 22 g | 40 à 300% |
| Hydroxyethylamidon | 0,36 g | 0,55 g | 0,5 à 20% |

Cette formulation permet d'obtenir après un mélange soutenu d'une durée de 3 mn, une pâte de consistance proche de la pâte à modeler.

La pâte est utilisable pendant environ 7 mn avant de commencer à prendre en masse.

La prise demande une durée de l'ordre de 15 minutes.

Le produit continue de durcir après ce temps jusqu'à obtenir une résistance de l'ordre de 480 Mpa après séchage. (fiche d'essai dureté)

Une légère augmentation de température est notée pendant la prise. Le produit peut atteindre une température de l'ordre de 40°C après un malaxage vigoureux. Ce qui reste très acceptable.

### EXEMPLE 2 : Moulage et fabrication de coins d'ostéotomie

Des déformations du genou telle que le « Genu varum » (talons joints, les faces internes des genoux restent séparées par une distance d'autant plus grande que le genu varum est plus prononcé) et le « Genu valgum » (à l'inverse du Genu varum, les genoux sont arcs boutés vers l'intérieur) entraînent très fréquemment une arthrose fémoro-tibiale localisée (interne ou externe).

Pour remédier à cela, une solution chirurgicale existe : l'ostéotomie d'ouverture. L'os tibial ou fémoral est sectionné pour pouvoir y introduire une cale ou coin d'ostéotomie (des plaques sont fixées pour éviter l'écrasement du coin), ce qui permet de modifier l'angle d'inclinaison entre le fémur et le tibia et de faire disparaître la déformation. Le positionnement de ce coin d'ostéotomie doit permettre une reconstruction osseuse, pour une conservation à long terme de la correction angulaire.

La présente invention permet au chirurgien de choisir pendant l'intervention chirurgicale, la taille du coin d'ostéotomie, pour obtenir l'angle de correction voulu, grâce à la présentation particulière de la composition de comblement osseux qui peut être moulée.

Le produit se présente sous la forme d'un kit qui peut contenir l'ensemble, ou simplement une partie, des composants suivants :
- moule : moule présentant des empreintes correspondantes à des coins d'ostéotomie de différentes tailles (variation des dimensions et/ou de la forme)
- produit de comblement osseux : La composition selon l'invention qui a un temps de durcissement d'environ 15 minute, par exemple la composition de l'exemple 1.

Après mélange, la composition obtenue est sous la forme d'une pâte malléable pendant environ 10 min. C'est pendant ce laps de temps que le produit peut être moulé à la forme d'un coin d'ostéotomie à l'aide d'un moule.

Après 15 minutes environ la pâte durcit. Le coin d'ostéotomie peut alors être démoulé et utilisé par le chirurgien.

Le temps de durcissement a été évalué par des essais comparatifs qui ont permis d'observer que le temps de mélange à une influence sur le temps de prise.

### EXEMPLE 3 : Essais de dureté

Des formulations selon l'invention préparées selon l'exemple 1 sont placées dans des cupules de scellage pendant au moins un heure, elles constituent les « éprouvettes »..

Le matériel pour tester la dureté est le suivant.
Machine de compression JFC-TC3 :
   Réglages:
      - Déplacement: 5mm/min
      - Force maximale : 500 N
      - Tige d'emboutissement cylindrique
      - Mesure de la pression (N/mm2) en fonction de la déformation (%)
Pieds à coulisse.

Le protocole suivit au cours de ces essais est le suivant :
• Mesure de l'épaisseur des « éprouvettes » à l'aide du pieds à coulisse :

| Identification | Épaisseur (mm) | | | |
|---|---|---|---|---|
| | 1^{ère} mesure | 2^{ème} mesure | 3^{ème} mesure | Moyenne |
| A | 8,23 | 7,88 | 7,95 | 8,02 |
| B | 7,70 | 7,60 | 7,55 | 7,62 |
| C | 7,22 | 7,82 | 8,05 | 7,70 |
| D | 7,60 | 7,36 | 7,32 | 7,43 |

• Mesure de la dureté des « éprouvettes » à l'aide de la machine JFC-TC3.

On obtient les résultats suivants :
• Mesure de l'épaisseur des « éprouvettes :

| Identification | Épaisseur (mm) | | | |
|---|---|---|---|---|
| | 1^{ère} mesure | 2^{éme} mesure | 3^{ème} mesure | Moyenne |
| A | 8,23 | 7,88 | 7,95 | 8,02 |
| B | 7,70 | 7,60 | 7,55 | 7,62 |
| C | 7,22 | 7,82 | 8,05 | 7,70 |
| D | 7,60 | 7,36 | 7,32 | 7,43 |

• Mesure de la dureté des « éprouvettes »:

| Identification | Contrainte (MPa) | | | | Déformation (mm) | | | |
|---|---|---|---|---|---|---|---|---|
| | 1^{ère} mesure | 2^{ème} mesure | 3^{ème} mesure | Moyenne | 1^{ère} mesure | 2^{ème} mesure | 3^{ème} mesure | Moyenne |
| A | 488,2 | 489,0 | 488,1 | 488,4 | 0,45 | 0,42 | 0,48 | 0,45 |
| B | 489,1 | 489,1 | 488,8 | 489,0 | 0,53 | 0,54 | 0,44 | 0,5 |
| C | 489,8 | 488,7 | 487,7 | 488,7 | 0,45 | 0,54 | 0,53 | 0,5 |
| D | 488,3 | 488,9 | 488,3 | 488,5 | 0,48 | 0,39 | 0,72 | 0,53 |
| | **Moyenne générale** | | | 488,7 | **Moyenne générale** | | | 0,5 |

Le produit, une fois durcit, résiste à une pression de l'ordre de 480 Mp.

### EXEMPLE 4 : OSTEOGENEICITE ET BOCOMPATIBLITE

### a) Implantation chez le lapin.

Les objectifs de cette implantation sont de vérifier que la composition selon l'invention n'induit pas une réaction inflammatoire impropre à la reconstruction osseuse, à savoir absence de fibrose installée et déut de reconstruction osseuse.

6 implantations chez des lapins adultes pendant 4 semaines de composition selon l'exemple 1 avec os allogénique ont été réalisées.

Les conclusions de l'essai ont été que la tolérance est compatible.

### b) Implantation sur moutons.

24 implantations sur moutons de composition selon l'invention avec os xénogénique ont été réalisées pendant 12 semaines (diamêtre 4,5 mm). Une étude histologique, densité osseuse et infiltration cellulaire, est ensuite réailisée.

La densité et l'homogénéité sont très satisfaisantes.

### c) Biocompatibilité.

Les tests de sensibilisation chez le cobaye (évaluation sur 38 cobayes, de cytotoxicité sur extrait (évaluation sur lignée cellulaire de souris), de toxicité systématique aiguê (évaluation sur 10 souris en IV, 10 en IP) et le test d'AMES (évaluation du pouvoir mutagène sur cinq souches de salmonelles) ont tous donnés des résultats conformes à la norme 10993.

## Revendications

1. Composition de comblement osseux sous forme de pâte malléable **caractérisée en ce qu'**elle comprend de la poudre d'os allogénique, du sulfate de calcium, un liant choisi parmi l'amidon ou un dérivé d'amidon et une solution de mélange.

2. Composition selon la revendication 1, **caractérisée en ce que** le dérivé d'amidon est choisi parmi l'hydroxyéthyl amidon, le carboxyméthyl amidon, l'amidon glycolique, l'amidon glycérol et l'amidon pregélatinisé.

3. Composition selon la revendication 1, **caractérisée en ce que** le dérivé d'amidon est l'hydroxyéthyl amidon.

4. Composition selon l'une quelconque des revendications précédentes **caractérisée en ce que** le sulfate de calcium est le sulfate de calcium hémihydraté, dont la forme cristalline caractérisée peut être alpha, bêta ou les deux.

5. Composition selon l'une quelconque des revendications précédentes **caractérisée en ce que** la poudre d'os allogénique est choisie parmi les poudres d'os humain ou non, déminéralisée ou non, ayant subi un traitement de viroinactivation, de granulométrie comprise entre 200 et 1600 µm.

6. Composition selon l'une quelconque des revendications précédentes **caractérisée en ce que** la solution de mélange est une solution aqueuse compatible physiologiquement choisie parmi un soluté isoton ique, du sérum physiologique ou un liquide physiologique comme du sérum ou du sang.

7. Composition selon l'une quelconque des revendications précédentes **caractérisée en ce que** la poudre d'os allogénique est incorporée dans des proportions comprises entre 0,1 et 80% v/v, le sulfate de calcium dans des proportions de 50 à 300% m/v, l'hydroxyéthyl amidon de 0,1 à 20% v/v et la solution de mélange de 0,3 à 80% vlv.

8. Utilisation d'une composition de comblement osseux sous forme de pâte malléable, pour la préparation d'un médicament pour le comblement des défects osseux, **caractérisée en ce que** la composition comprend de la poudre d'os allogénique, du sulfate de calcium, un liant choisi parmi l'amidon ou un dérivé d'amidon et une solution de mélange.

9. Kit constitué d'un contenant comprenant une composition comprenant de la poudre d'os allogénique, du sulfate de calcium, un liant choisi parmi l'amidon ou un dérivé d'amidon et un contenant comprenant la solution de mélange.

10. Kit selon la revendication précédente **caractérisé en ce qu'**il comprend en outre des moules pour coins d'ostéotomie.

## Claims

1. A bone filler composition in malleable paste form, **characterized in that** it comprises allogeneic bone powder, calcium sulphate, a binder chosen from among starch or a starch derivative and a mixing solution.

2. The composition according to claim 1, **characterized in that** the starch derivative is chosen from among hydroxyethyl starch, carboxymethyl starch, glycol starch, glycerol starch and pre-gelatinized starch.

3. The composition according to claim 1, **characterized in that** the starch derivative is hydroxyethyl starch.

4. The composition according to any of the preceding claims, **characterized in that** the calcium sulphate is semi-hydrated calcium sulphate whose characterized crystalline form may be alpha, beta or both.

5. The composition according to any of the preceding claims, **characterized in that** the allogeneic bone powder is chosen from among human or non-human, demineralized or non-demineralized bone powders subjected to viral inactivation treatment and having a particle size of between 200 and 1600 µm.

6. The composition according to any of the preceding claims, **characterized in that** the mixing solution is a physiologically compatible aqueous solution chosen from among an isotonic solution, physiological saline solution or a physiological liquid such as serum or blood.

7. The composition according to any of the preceding claims, **characterized in that** the allogeneic bone powder is incorporated in proportions of between 0.1 and 80 % v/v, the calcium sulphate in proportions of 50 to 300 % w/v, the hydroxyethyl starch from 0.1 to 20 % v/v and the mixing solution from 0.3 to 80 % v/v.

8. The use of a bone filler composition in malleable paste form for the preparation of a medication to fill bone defects, **characterized in that** the composition comprises allogeneic bone powder, calcium sulphate, a binder chosen from among starch or a starch derivative and a mixing solution.

9. A kit formed of a container containing a composition comprising allogeneic bone powder, calcium sulphate, a binder chosen from among starch or a starch derivative, and a container containing the mixing solution.

10. The kit according to the preceding claim, **characterized in that** it further comprises moulds for osteotomy wedges.

## Patentansprüche

1. Knochenersatzmaterial-Zusammensetzung in Form einer formbaren Paste, **dadurch gekennzeichnet, dass** sie allogenes Knochenpulver, Calciumsulfat, ein Bindemittel, das aus der Stärke oder einem Stärkederivat ausgewählt ist und eine Mischlösung umfasst.

2. Zusammensetzung nach Anspruch 1, **dadurch gekennzeichnet, dass** das Stärkederivat aus der Hydroxyethylstärke, der Carboxymethylstärke, der Glycolstärke, der Glycerolstärke und der vorgelatinierten Stärke ausgewählt ist.

3. Zusammensetzung nach Anspruch 1, **dadurch gekennzeichnet, dass** das Stärkederivat die Hydroxyethylstärke ist.

4. Zusammensetzung nach einem der vorangehenden Ansprüche, **dadurch gekennzeichnet, dass** das Calciumsulfat das hemihydratisierte Calcium ist, dessen charakterisierte kristalline Form alpha, beta oder beides sein kann.

5. Zusammensetzung nach einem der vorangehenden Ansprüche, **dadurch gekennzeichnet, dass** das allogene Knochenpulver aus den humanen oder nicht humanen, demineralisierten oder nicht demineralisierten Knochenpulvern ausgewählt ist, die einer Virusinaktivierungsbehandlung unterzogen wurden, mit einer Korngröße zwischen 200 und 1600 µm inklusive.

6. Zusammensetzung nach einem der vorangehenden Ansprüche, **dadurch gekennzeichnet, dass** die Mischlösung eine wässrige Lösung ist, die physiologisch kompatibel ist, die aus einer isotonischen Lösung, physiologischem Serum oder einer physiologischen Flüssigkeit wie Serum oder Blut ausgewählt ist.

7. Zusammensetzung nach einem der vorangehenden Ansprüche, **dadurch gekennzeichnet, dass** das allogene Knochenpulver in Verhältnissen zwischen 0,1 und 80 % v/v, das Calciumsulfat in Verhältnissen von 50 bis 300 % m/v, die Hydroxyethylstärke von 0,1 bis 20 % v/v und die Mischlösung von 0,3 bis 80 % inkorporiert wird.

8. Verwendung einer Knochenersatzmaterial-Zusammensetzung in Form einer formbaren Paste für die Herstellung eines Arzneimittels zum Auffüllen von Knochendefekten, **dadurch gekennzeichnet, dass** die Zusammensetzung allogenes Knochenpulver, Calciumsulfat, ein Bindemittel, das aus der Stärke oder einem Stärkederivat ausgewählt ist, und eine Mischlösung umfasst.

9. Kit, das aus einem Behältnis besteht, das eine Zusammensetzung umfasst, die allogenes Knochenpulver, Calciumsulfat, ein Bindemittel, das aus der Stärke oder einem Stärkederivat ausgewählt ist, und einen Behälter, der die Mischlösung umfasst, umfasst.

10. Kit nach vorangehendem Anspruch, **dadurch gekennzeichnet, dass** es ferner Formen für Osteotomiekeile umfasst.
